# EUROPEAN PATENT SPECIFICATION

(11) **EP 4 529 569 B1**
(45) Date of publication and mention of the grant of the patent: **07.10.2026**
(21) Application number: 24775400.5
(22) Date of filing: 14.03.2024
(51) Int. Cl.: G01N 21/64, G01N 15/14, G16B 30/00, C12Q 1/6851, G02B 21/16, G02B 27/00, C12Q 1/6869

(54) **METHOD FOR EXTENDED DEPTH OF FIELD**
VERFAHREN FÜR ERWEITERTE FELDTIEFE
PROCÉDÉ DE PROFONDEUR DE CHAMP ÉTENDUE

(30) Priority: 17.03.2023 US 202363452835 P
(43) Date of publication of application: 02.04.2025
(73) Proprietor: ILLUMINA, INC., San Diego, CA 92122 (US)
(72) Inventor: GAU, Jeffrey, San Diego, CA 92122 (US); PINTO, Joseph, San Diego, CA 92122 (US); DUTTA, Anindita, San Diego, CA 92122 (US); EVANS, Geraint, San Diego, CA 92122 (US); WANG, Yina, San Diego, CA 92122 (US); PRINCE, Simon, San Diego, CA 92122 (US); HONG, Stanley, San Diego, CA 92122 (US)
(74) Representative: Murgitroyd & Company
(86) International application number: PCT/US2024/019870
(87) International publication number: WO 2024/196690

(56) References cited:
- WO-A1-2022/029768
- US-A1- 2017 146 706
- US-A1- 2017 146 706
- US-A1- 2019 056 581
- US-A1- 2019 087 948
- US-A1- 2021 222 239
- US-A1- 2022 267 842
- US-A1- 2022 267 842
- TINGYU ZHAO ET AL: "Optimization of wavefront-coded infinity-corrected microscope systems with extended depth of field", BIOMEDICAL OPTICS EXPRESS, vol. 4, no. 8, 31 July 2013 (2013-07-31), United States, pages 1464, XP055356287, ISSN: 2156-7085, DOI: 10.1364/BOE.4.001464
- BO SHUANG, WENXIAO WANG, HAO SHEN, LAWRENCE J. TAUZIN, CHARLOTTE FLATEBO, JIANBO CHEN, NICHOLAS A. MORINGO, LOGAN D. C. BISHOP, KE: "Generalized recovery algorithm for 3D super-resolution microscopy using rotating point spread functions", SCIENTIFIC REPORTS, vol. 6, no. 1, 1 November 2016 (2016-11-01), XP055623099, DOI: 10.1038/srep30826
- MATSUMOTO NAOYA, KONNO ALU, INOUE TAKASHI, WATANABE KOYO, OKAZAKI SHIGETOSHI: "Amplitude-modulation-type multi-ring mask for two-photon excitation scanning microscopy", OSA CONTINUUM, vol. 4, no. 6, 15 June 2021 (2021-06-15), pages 1696, XP093233469, ISSN: 2578-7519, DOI: 10.1364/OSAC.419804
- SHENG LIU, HONG HUA: "Extended depth-of-field microscopic imaging with a variable focus microscope objective", OPTICS EXPRESS, SPIE PRESS, vol. 19, no. 1, 3 January 2011 (2011-01-03), pages 353 - 362, XP055414801, DOI: 10.1364/OE.19.000353
- TINGYU ZHAO, THOMAS MAUGER, GUOQIANG LI: "Optimization of wavefront-coded infinity-corrected microscope systems with extended depth of field", BIOMEDICAL OPTICS EXPRESS, OPTICAL SOCIETY OF AMERICA, UNITED STATES, vol. 4, no. 8, 1 August 2013 (2013-08-01), United States , pages 1464, XP055356287, ISSN: 2156-7085, DOI: 10.1364/BOE.4.001464
- LIAO MEIHUA, LU DAJIANG, PEDRINI GIANCARLO, OSTEN WOLFGANG, SITU GUOHAI, HE WENQI, PENG XIANG: "Extending the depth-of-field of imaging systems with a scattering diffuser", SCIENTIFIC REPORTS, NATURE PUBLISHING GROUP, US, vol. 9, no. 1, US , XP093233470, ISSN: 2045-2322, DOI: 10.1038/s41598-019-43593-w
- SCHNEIDER MAGDALENA C.; HINTERER FABIAN; JESACHER ALEXANDER; SCHÜTZ GERHARD J.: "Interactive simulation and visualization of point spread functions in single molecule imaging", OPTICS COMMUNICATIONS, ELSEVIER, AMSTERDAM, NL, vol. 560, 12 March 2024 (2024-03-12), AMSTERDAM, NL , XP087497337, ISSN: 0030-4018, DOI: 10.1016/j.optcom.2024.130463

## Description

### BACKGROUND

The subject matter discussed in this section should not be assumed to be prior art merely as a result of its mention in this section. Similarly, a problem mentioned in this section or associated with the subject matter provided as background should not be assumed to have been previously recognized in the prior art. The subject matter in this section merely represents different approaches, which in and of themselves may also correspond to implementations of the claimed technology.

A primary driver of optical signal to noise ratio is a point spread function (PSF), and the PSF will degrade as defocus on an object being image decreases. Thus, technology for improving focus during sequencing may decrease noise, and thereby allow for increased accuracy in base calling, lower cost through higher numerical aperture objectives, and increased throughput through larger field of view. Accordingly, there is a need for improvements in technology for improving focus, such as through extending the depth of field of imaging components used in sequencers. US 2022/267842 A1 discloses examples of fluorescence imaging system designs. US 2017/146706 A1 discloses an example of an optical element. Zhao et al., 2013 (DOI: 10.1364/BOE.4.001464) discloses a practical wavefront-coded infinity-corrected microscope system and optimized methods for use. WO 2022/029768 A1 discloses a method, system and computer readable medium configured to determine the presence of a molecule in a sample. US 10416428 B2 discloses systems and methods for improving accuracy of focus tracking in optical systems and reducing latency associated with focus tracking in optical scanners.

### SUMMARY

Examples disclosed herein are directed to techniques for extending the depth of field of sequencers.

An implementation described herein relates to a nucleic acid sequencer comprising: a flow delivery module configured to add fluorescent tags to nucleic acid sites by flowing reagents through a flow cell comprising the nucleic acid sites; and a detection system configured to detect the fluorescent tags, the detection system comprising an objective lens, a set of detection pixels, and a mask disposed between the objective lens and the set of detection pixels.

In some implementations described herein, such as described in the second paragraph of this summary, the mask is a phase mask.

In some implementations described herein, such as described in the second paragraph of this summary, the mask is an amplitude mask.

In some implementations described herein, such as described in any of the second through fourth paragraphs of this summary, the detection system has a numerical aperture of about 1.1 or greater, and depth of field of greater than +/- 300 nm.

In some implementations described herein, such as described in an of the second through fourth paragraphs of this summary, the depth of field of the detection system is greater than or equal to 1000 nm.

In some implementations described herein, such as described in any of the second through sixth paragraphs of this summary, the mask is a deformable mirror or a spatial light modulator.

In some implementations described herein, such as described in any of the second through seventh paragraphs of this summary, the mask comprises two or more materials, wherein each material of the two or more materials is a glass having a difference in dispersion greater than about 5% from the dispersion of every other material from the two or more materials.

In some implementations described herein, such as described in any of the second through ninth paragraphs of this summary, the objective lens is comprised by an objective lens assembly within the detection system, and the mask is disposed between the objective lens and a tube lens within the objective lens assembly.

An implementation in accordance with the present invention relates to a method as defined in claim 1.

In some implementations such as described in the tenth paragraph of this summary, the first result is a first point spread function (PSF) indicating how light emitted by the sample at the nucleic acid site is detected by the set of detection pixels after passing through the objective lens and the mask when the objective lens is out of focus by a target defocus amount; and the second result is a second PSF indicating how light emitted by the sample at the nucleic acid site is detected by the set of detection pixels when the sample at the nucleic acid site is focused on a surface of the sample.

In some implementations such as described in the eleventh paragraph of this summary, the nucleic acid sequencer is configured to detect light signals emitted from multiple surfaces of a multi-surface flowcell; updating the mask based on the determined discrepancy comprises applying a loss function, wherein the piecewise loss function comprises: a first loss component configured to ensure that signals from a first surface of the multi-surface flowcell are in focus; and a second loss component configured to ensure that signals from a second surface of the multi-surface flowcell are out of focus.

In some implementations such as described in the tenth paragraph of this summary, the mask is an amplitude mask; the sample at the nucleic acid site comprises a plurality of clusters; the set of optimization steps comprises: separating the plurality of clusters into a first set of clusters and a second set of clusters; and updating the mask based on the determined discrepancy comprises determining a loss based on a mean and a standard deviation of the first set of clusters, and a mean and a standard deviation of the second set of clusters.

In some implementations such as described in the tenth paragraph of this summary, the first result is a sequence of base calls generated by a base calling algorithm based on light emitted from the sample at the nucleic acid site and detected by the set of detection pixels after passing through objective lens and the mask; and the second result is a sequence of base calls for a reference genome.

In some implementations such as described in the fourteenth paragraph of this summary, the base calling algorithm comprises a machine learning model; and the set of updating tasks comprises updating the machine learning model based on the discrepancy between the first result and the second result.

In some implementations such as described in the tenth paragraph of this summary, the nucleic acid sequencer is configured to detect light signals in a plurality of wavelengths; the first result is a result of passing a first wavelength of light emitted by the sample at the nucleic acid site through the objective lens and the mask; and the set of optimization steps comprises: determining a third result, wherein the third result is a result of passing a second wavelength of light emitted by the sample at the nucleic acid site through the objective lens and a second mask, wherein the objective lens is focused on the plane which is separated from the nucleic acid site by the target defocus amount, and wherein the second mask is disposed between the objective lens and a second set of detection pixels; determining whether there is a discrepancy between the second result with and the third result; and based on determining there is a discrepancy between the second result and the third result, performing a second set of updating tasks comprising updating the second mask based on the determined discrepancy between the second result and the third result.

In some implementations such as described in the tenth paragraph of this summary, the mask is a phase mask.

In some implementations such as described in the tenth paragraph of this summary, on each repetition of the set of optimization steps: determining the first result is performed a plurality of times, wherein each time from the plurality of times, the target defocus amount is different from the target defocus amount on each other time from the plurality of times; performing the set of updating tasks is performed a set of times, wherein each time the set of updating tasks is performed, a loss is calculated which corresponds to the target defocus amount from a different time from the plurality of times.

In some implementations such as described in the eighteenth paragraph of this summary, on each repetition of the set of optimization steps, the plurality of times the first result is determined has a cardinality less than the set of times the set of updating tasks is performed.

In some implementations such as described in the nineteenth paragraph of this summary, on each repetition of the set of optimization tasks: the set of updating tasks comprises determining the target defocus resulting in the largest discrepancies between the first result and the second result; and each loss which is calculated during performance of the set of updating tasks corresponds to a target defocus amount which results in a greater discrepancy between the first result and the second result than any target defocus amount which does not correspond to a loss calculated during that repetition of the set of optimization tasks.

Other features and aspects of the disclosed technology will become apparent from the following detailed description, taken in conjunction with the accompanying drawings, which illustrate, by way of example, the features in accordance with examples of the disclosed technology. The summary is not intended to limit the scope of any protection provided by this document or any related document, which scope is defined by the respective document's claims and equivalents.

It should be appreciated that all combinations of the foregoing concepts (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

### BRIEF DESCRIPTION OF THE DRAWINGS

The present disclosure, in accordance with one or more various examples, is described in detail with reference to the following figures. The figures are provided for purposes of illustration only and merely depict typical or example implementations.
FIG. 1 depicts a schematic view of another example of a system that may be used to provide biological or chemical analysis..
FIG. 2 is block diagram illustrating an example two-channel, line-scanning modular optical imaging system that may be implemented in particular implementations.
FIG. 3 illustrates an example configuration of a patterned sample that may be imaged in accordance with implementations disclosed herein.
FIG. 4 illustrates an optical configuration for extending usable depth of field.
FIG. 5 illustrates impacts of optical masks implemented based on this disclosure.
FIG. 6 illustrates a method by which an optical mask may be designed.
FIG. 7 illustrates a method by which an optical mask may be designed.
FIG. 8 illustrates an end to end design process.
FIG. 9 illustrates cross sections of optimized and simplified phase masks.
FIG. 10 illustrates how phase masks can be created using combinations of materials.
FIG. 11 illustrates an example computing module that may be used to implement various features of implementations described in the present disclosure.

The figures are not exhaustive and do not limit the present disclosure to the precise form disclosed.

### DETAILED DESCRIPTION

As used herein to refer to a sample, the term "spot" or "feature" is intended to mean a point or area in a pattern that may be distinguished from other points or areas according to relative location. An individual spot may include one or more molecules of a particular type. For example, a spot may include a single target nucleic acid molecule having a particular sequence or a spot may include several nucleic acid molecules having the same sequence (and/or complementary sequence, thereof).

As used herein to refer to a spot or feature in connection with a direction, the term "pitch" is intended to mean the separation of the spot or feature from other spots or features in the direction. For example, if a sample container has an array of features which are separated from each other by 650 nm in the direction that the container would be moved during imaging, then the "pitch" of the features in that direction may be referred to as being 650 nm.

As used herein, the term "xy plane" is intended to mean a 2 dimensional area defined by straight line axes x and y in a Cartesian coordinate system. When used in reference to a detector and an object observed by the detector, the area may be further specified as being orthogonal to the direction of observation between the detector and object being detected. When used herein to refer to a line scanner, the term "y direction" refers to the direction of scanning.

As used herein, the term "z coordinate" is intended to mean information that specifies the location of a point, line or area along an axes that is orthogonal to an xy plane. In particular implementations, the z axis is orthogonal to an area of an object that is observed by a detector. For example, the direction of focus for an optical system may be specified along the z axis.

As used herein, the term "scan a line" is intended to mean detecting a 2-dimensional cross-section in an xy plane of an object, the cross-section being rectangular or oblong, and causing relative movement between the cross-section and the object. For example, in the case of fluorescence imaging an area of an object having rectangular or oblong shape may be specifically excited (at the exclusion of other areas) and/or emission from the area may be specifically acquired (at the exclusion of other areas) at a given time point in the scan.

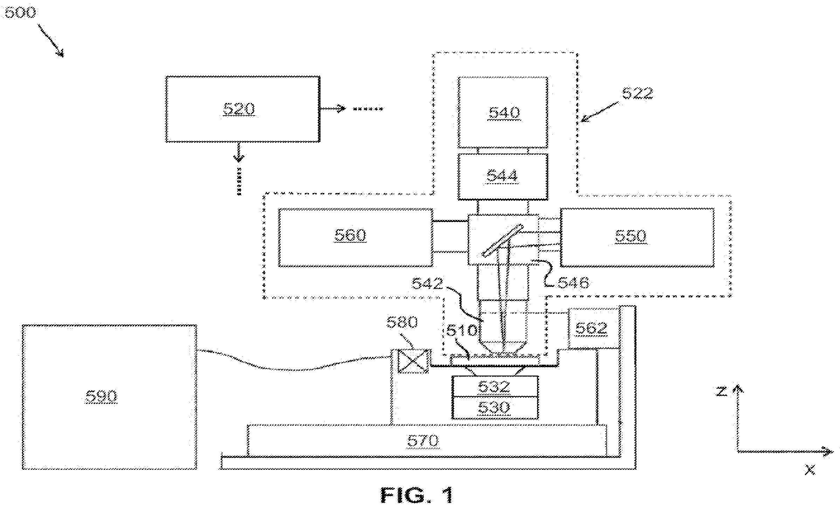

Discussed herein is technology which may improve focus during sequencing by extending the depth of field in which a sample can be imaged. FIG. 1 illustrates a schematic diagram of another example of a system **500** that may be used to perform an analysis on one or more samples of interest. System **500** is configured to perform a large number of parallel reactions within a flow cell **510.** Flow cell **510** may thus include one or more flow channels that receive a solution from system **500** and direct the solution toward reaction sites of flow cell **510.**

System **500** includes a system controller **520** that may communicate with the various components, assemblies, and sub-systems of the system **500.** An imaging assembly **522** of system **500** includes a light emitting assembly **550** that emits light that reaches reaction sites on flow cell **510.** Light emitting assembly **550** may include an incoherent light emitter (e.g., emit light beams output by one or more excitation diodes), or a coherent light emitter such as emitter of light output by one or more lasers or laser diodes. In some implementations, light emitting assembly **550** may include a plurality of different light sources (not shown), each light source emitting light of a different wavelength range. Some versions of light emitting assembly **550** may also include one or more collimating lenses (not shown), a light structuring optical assembly (not shown), a projection lens (not shown) that is operable to adjust a structured beam shape and path, epifluorescence microscopy components, and/or other components. Although system **500** is illustrated as having a single light emitting assembly **550,** multiple light emitting assemblies **550** may be included in some other implementations.

In the present example, the light from light emitting assembly **550** is directed by dichroic mirror assembly **546** through an objective lens assembly **542** onto a sample of a flow cell **510,** which is positioned on a motion stage **570.** In the case of fluorescent microscopy of a sample, a fluorescent element associated with the sample of interest fluoresces in response to the excitation light, and the resultant light is collected by objective lens assembly **542** and is directed to an image sensor of camera system **540** to detect the emitted fluorescence. In some implementations, a tube lens assembly may be positioned between the objective lens assembly **542** and the dichroic mirror assembly **546** or between the dichroic mirror **546** and the image sensor of the camera system **540.** A moveable lens element may be translatable along a longitudinal axis of the tube lens assembly to account for focusing on an upper interior surface or lower interior surface of the flow cell **510** and/or spherical aberration introduced by movement of the objective lens assembly **542.**

In the present example, a filter switching assembly **544** is interposed between dichroic mirror assembly **546** and camera system **540.** Filter switching assembly **544** includes one or more emission filters that may be used to pass through particular ranges of emission wavelengths and block (or reflect) other ranges of emission wavelengths. For example, emission filters may be used to direct different wavelength ranges of emitted light to different image sensors of the camera system **540** of imaging assembly **522.** For instance, the emission filters may be implemented as dichroic mirrors that direct emission light of different wavelengths from flow cell **510** to different image sensors of camera system **540.** In some variations, a projection lens is interposed between filter switching assembly **544** and camera system **540.** Filter switching assembly **544** may be omitted in some versions.

System **500** further includes a fluid delivery assembly **590** that may direct the flow of reagents (e.g., fluorescently labeled nucleotides, buffers, enzymes, cleavage reagents, etc.) to (and through) flow cell **510** and waste valve **580.** System **500** of the present example also includes a temperature station actuator **530** and heater/cooler **532** that may optionally regulate the temperature of conditions of the fluids within the flow cell **510.** In some implementations, the heater/cooler **532** may be fixed to sample stage **570,** upon which the flow cell **510** is placed, and/or may be integrated into sample stage **570.**

Flow cell **510** may be removably mounted on sample stage **570,** which may provide movement and alignment of flow cell **510** relative to objective lens assembly **542.** Sample stage **570** may have one or more actuators to allow sample stage **570** to move in any of three dimensions. For example, actuators may be provided to allow sample stage **570** to move in the x, y, and z directions relative to objective lens assembly **542,** tilt relative to objective lens assembly **542,** and/or otherwise move relative to objective lens assembly **542.** Movement of sample stage **570** may allow one or more sample locations on flow cell **510** to be positioned in optical alignment with objective lens assembly **542.** Movement of sample stage **570** relative to objective lens assembly **542** may be achieved by moving sample stage **570** itself, by moving objective lens assembly **542,** by moving some other component of imaging assembly **522,** by moving some other component of system **500,** or any combination of the foregoing. For instance, in some implementations, the sample stage **570** may be actuatable in the x and y directions relative to the objective lens assembly **542** while a focus component **562** or z-stage may move the objective lens assembly **542** along the z direction relative to the sample stage **570.**

In some implementations, a focus component **562** may be included to control positioning of one or more elements of objective lens assembly **542** relative to the flow cell **510** in the focus direction (e.g., along the z-axis or z-dimension). Focus component **562** may include one or more actuators physically coupled to the objective lens assembly **542,** the optical stage, the sample stage **570,** or a combination thereof, to move flow cell **510** on sample stage **570** relative to the objective lens assembly **542** to provide proper focusing for the imaging operation. In the present example, the focus component **562** utilizes a focus tracking module **560** that is configured to detect a displacement of the objective lens assembly **542** relative to a portion of the flow cell **510** and output data indicative of an in-focus position to the focus component **562** or a component thereof or operable to control the focus component **562,** such as controller **520,** to move the objective lens assembly **542** to position the corresponding portion of the flow cell **510** in focus of the objective lens assembly **542.**

In some implementations, an actuator of focus component **562** or for sample stage **570** may be physically coupled to objective lens assembly **542,** the optical stage, sample stage **570,** or a combination thereof, such as, for example, by mechanical, magnetic, fluidic, or other attachment or contact directly or indirectly to or with the stage or a component thereof. The actuator of focus component **562** may be configured to move objective lens assembly **542** in the z-direction while maintaining sample stage **570** in the same plane (e.g., maintaining a level or horizontal attitude, perpendicular to the optical axis). In some implementations, sample stage **570** includes an x direction actuator and a y direction actuator to form an x-y stage. Sample stage **570** may also be configured to include one or more tip or tilt actuators to tip or tilt sample stage **570** and/or a portion thereof, to account for any slope in its surfaces.

Camera system **540** may include one or more image sensors to monitor and track the imaging (e.g., sequencing) of flow cell **510.** Camera system **540** may be implemented, for example, as a CCD or CMOS image sensor camera, but other image sensor technologies (e.g., active pixel sensor) may be used. By way of further example only, camera system **540** may include a dual-sensor time delay integration (TDI) camera, a single-sensor camera, a camera with one or more two-dimensional image sensors, and/or other kinds of camera technologies. In various embodiments, one or more image sensors may be positioned under flow cell **510,** such as within the sample stage **570** or below the sample stage **570;** or may even be integrated into flow cell **510.**

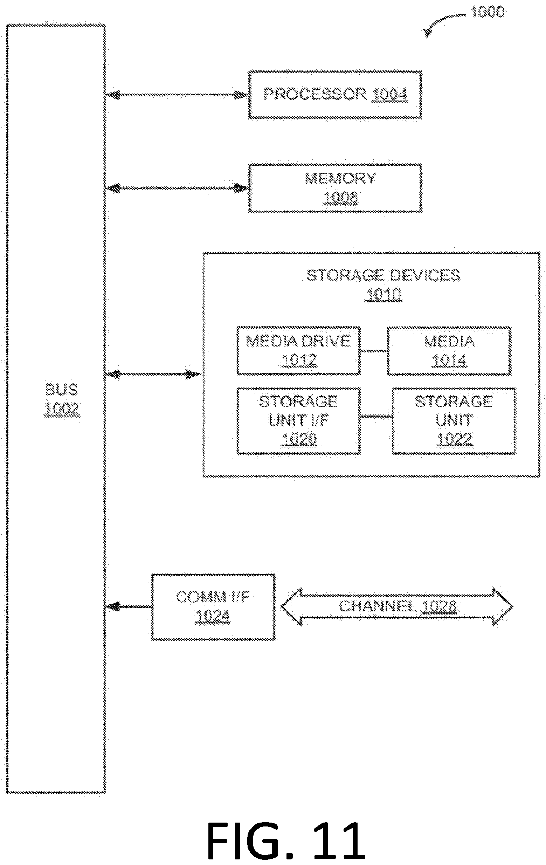

Although not illustrated, a controller, which may be implemented as a computing module such as discussed infra in the context of FIG. 11, may be provided to control the operation of the scanning system. The controller may be implemented to control aspects of system operation such as, for example, focusing, stage movement, and imaging operations. In various implementations, the controller may be implemented using hardware, algorithms (e.g., machine executable instructions), or a combination of the foregoing. For example, in some implementations the controller may include one or more CPUs or processors with associated memory. As another example, the controller may comprise hardware or other circuitry to control the operation, such as a computer processor and a non-transitory computer readable medium with machine-readable instructions stored thereon. For example, this circuitry may include one or more of the following: field programmable gate array (FPGA), application specific integrated circuit (ASIC), programmable logic device (PLD), complex programmable logic device (CPLD), a programmable logic array (PLA), programmable array logic (PAL) or other similar processing device or circuitry. As yet another example, the controller may comprise a combination of this circuitry with one or more processors.

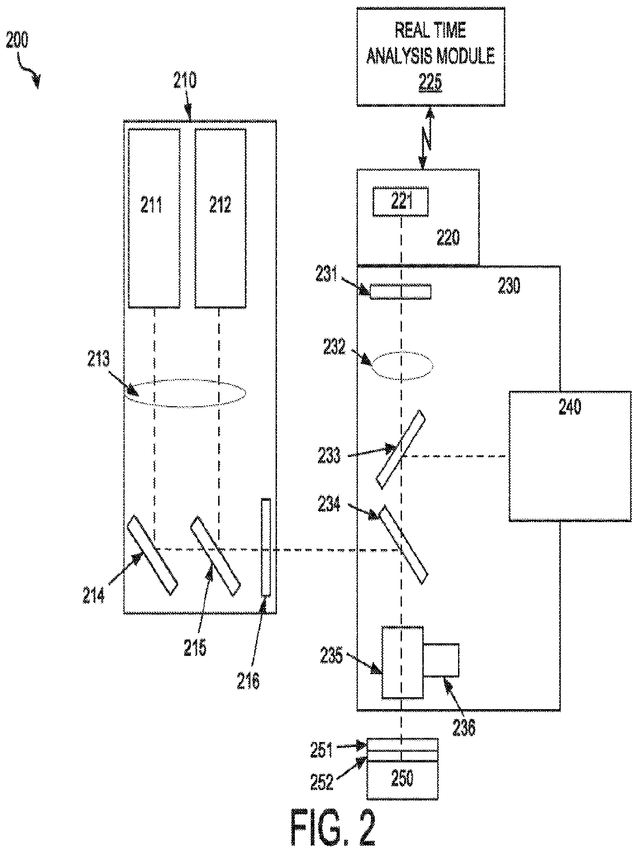

Other imaging systems may also be used when implementing the disclosed technology. For example, FIG. 2 is block diagram illustrating an example two-channel, line-scanning modular optical imaging system **200** in which aspects of the disclosed technology may be implemented. In some implementations, system **200** may be used for the sequencing of nucleic acids. Applicable techniques include those where nucleic acids are attached at fixed locations in an array (e.g., the wells of a flow cell) and the array is imaged repeatedly while in motion relative to the field of view of a camera in the imaging system **200.** In such implementations, system **200** may obtain images in two different color channels, which may be used to distinguish a particular nucleotide base type from another. More particularly, system **200** may implement a process referred to as "base calling," which generally refers to a process of a determining a base call (e.g., adenine (A), cytosine (C), guanine (G), or thymine (T)) for a given spot location of an image at an imaging cycle. During two-channel base calling, image data extracted from two images may be used to determine the presence of one of four base types by encoding base identity as a combination of the intensities of the two images. For a given spot or location in each of the two images, base identity may be determined based on whether the combination of signal identities is [on, on], [on, off], [off, on], or [off, off].

Referring again to imaging system **200,** the system includes a line generation module (LGM) **210** with two light sources, **211** and **212,** disposed therein. Light sources **211** and **212** may be coherent light sources such as laser diodes which output laser beams. Light source **211** may emit light in a first wavelength (e.g., a red color wavelength), and light source **212** may emit light in a second wavelength (e.g., a green color wavelength). The light beams output from laser sources **211** and **212** may be directed through a beam shaping lens or lenses **213.** In some implementations, a single light shaping lens may be used to shape the light beams output from both light sources. In other implementations, a separate beam shaping lens may be used for each light beam. In some examples, the beam shaping lens is a Powell lens, such that the light beams are shaped into line patterns. The beam shaping lenses of LGM **210** or other optical components imaging system may shape the light emitted by light sources **211** and **212** into a line patterns (e.g., by using one or more Powel lenses, or other beam shaping lenses, diffractive or scattering components).

LGM **210** may further include mirror **214** and semi-reflective mirror **215** to direct the light beams through a single interface port to an emission optics module (EOM) **230.** The light beams may pass through a shutter element **216.** EOM **230** may include objective **235** and a z-stage **236** which moves objective lens **235** longitudinally closer to or further away from a target **250.** For example, target **250** may include a liquid layer **252** and a translucent cover plate **251,** and a biological sample may be located at an inside surface of the translucent cover plate as well an inside surface of the substrate layer located below the liquid layer. The z-stage **236** may then move the objective as to focus the light beams onto either inside surface of the flow cell (e.g., focused on the biological sample). Similarly, in some implementations, the target **250** may be mounted on, or include a stage movable in the xy plane relative to the objective lens **235.** The biological sample may be DNA, RNA, proteins, or other biological materials responsive to optical sequencing as known in the art.

EOM **230** may include semi-reflective mirror **233** to reflect a focus tracking light beam emitted from a focus tracking module (FTM) **240** onto target **250,** and then to reflect light returned from target **250** back into FTM **240.** FTM **240** may include a focus tracking optical sensor to detect characteristics of the returned focus tracking light beam and generate a feedback signal to optimize focus of objective **235** on target **250.**

EOM **230** may also include semi-reflective mirror **234** to direct light through objective lens **235,** while allowing light returned from target **250** to pass through. In some implementations, EOM **230** may include a tube lens **232.** Light transmitted through tube lens **232** may pass through filter element **231** and into camera module (CAM) **220.** CAM **220** may include one or more optical sensors **221** to detect light emitted from the biological sample in response to the incident light beams (e.g., fluorescence in response to red and green light received from light sources **211** and **212**).

Output data from the sensors of CAM **220** may be communicated to a real time analysis module **225.** Real time analysis module, in various implementations, executes computer readable instructions for analyzing the image data (e.g., image quality scoring, base calling, etc.), reporting or displaying the characteristics of the beam (e.g., focus, shape, intensity, power, brightness, position) to a graphical user interface (GUI), etc. These operations may be performed in real-time during imaging cycles to minimize downstream analysis time and provide real time feedback and troubleshooting during an imaging run. In implementations, real time analysis module may be a computing device (e.g., computing device **1000**) that is communicatively coupled to and controls imaging system **200.** In implementations further described below, real time analysis module **225** may additionally execute computer readable instructions for controlling illumination of the target **250** and optionally for integrating data gathered during multiple exposures of the optical sensor(s) **221** into an image.

It is possible that still other types of imaging systems may be used with the disclosed technology. For example, the disclosed technology may be used in line scan systems, but it may also be used in area scan systems, in which a two dimensional pixel array is used to capture light from a sample in a step and shoot style procedure. Accordingly, the illustrations and discussions related to line scan systems should be understood as being illustrative only, and should not be treated as limiting.

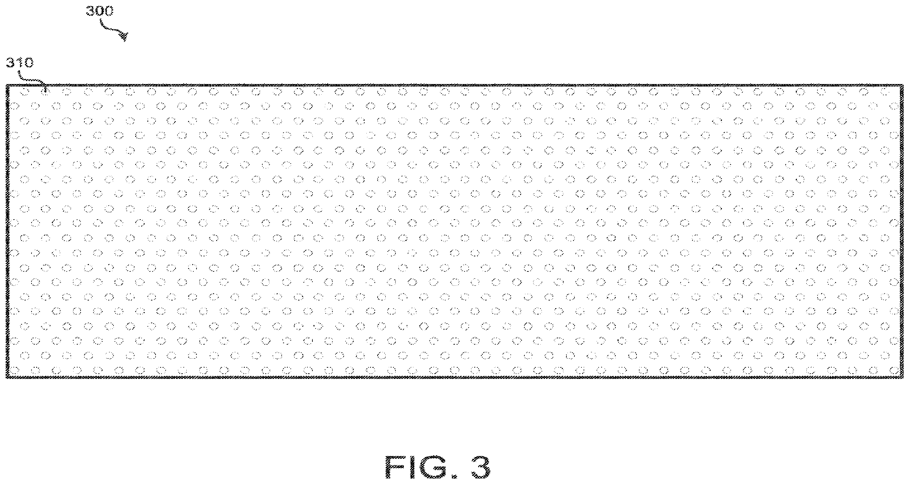

FIG. 3 illustrates an example configuration of a sample container **300** that may be imaged in accordance with implementations disclosed herein. In this example, sample container **300** is patterned with a hexagonal array of ordered spots **310** that may be simultaneously imaged during an imaging run. Although a hexagonal array is illustrated in this example, in other implementations the sample container may be patterned using a rectilinear array, a circular array, an octagonal array, or some other array pattern. For ease of illustration, sample container **300** is illustrated as having tens to hundreds of spots **310.** However, as may be appreciated by one having skill in the art, sample container **300** may have thousands, millions, or billions of spots **310** that are imaged. Moreover, in some instances, sample container **300** may be a multi-plane sample comprising multiple planes (perpendicular to focusing direction) of spots **310** that are sampled during an imaging run.

In a particular implementation, sample container **300** may be a flow cell patterned with millions or billions of wells that are divided into lanes. In this particular implementation, each well of the flow cell may contain biological material that is sequenced using sequencing by synthesis.

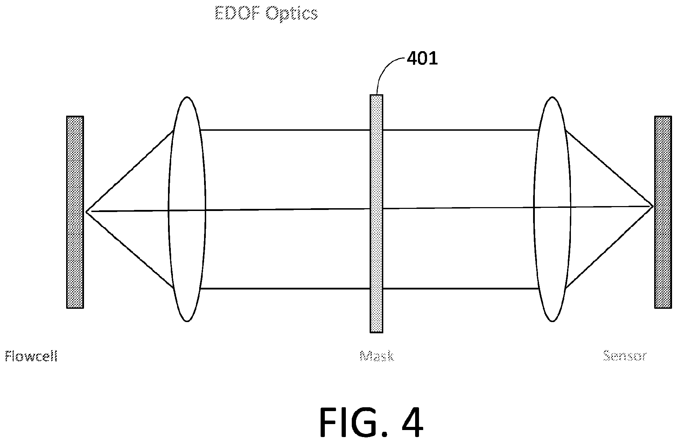

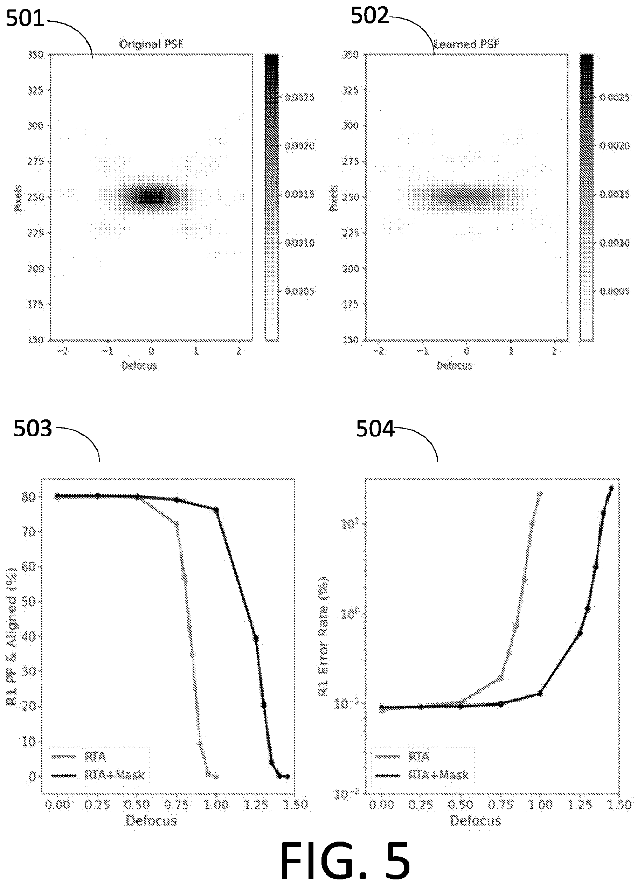

Turning now to FIG. 4, that figure illustrates a simplified optical configuration for extending usable depth of field in which a mask **401** is disposed at the exit pupil plane between an objective and a sensor, such as within objective lens assembly **542** of FIG. 1, or objective **235** and sensors **221** of FIG. 2. In different variations, such a mask may be disposed at any point on an optical path between the objective lens and the sensor, such as between an objective lens **235** and a tube lens **232** in a configuration such as shown in FIG. 2. In operation, this mask **401** will modify the field at the exit plane which, in turn, will modify the point spread function, and will allow for improved focus throughout a broader depth of field than may otherwise be feasible. FIG. 5 illustrates the impact that such a mask can have, with a first graph **501** showing a point spread function in a sequencer without a mask, and a second graph **502** showing a point spread function in a sequencer which includes a mask. As indicated in those graphs, including a mask can result in a sequencer having a distinguishable point spread function over a larger range of focus values - e.g., +/- 1000 nm versus +/- 600 nm, as illustrated (or +/- 300 nm versus +/- 190 nm in a system with a numerical aperture of greater than or equal to about 1.1, such as a system with a numerical aperture of 1.1 precisely, a system with a numerical aperture of 1.2, a system with a numerical aperture of 1.3, etc.). This demonstrates that inclusion of a mask can successfully extend the sequencer's depth of field by providing a dimmer (but still distinguishable) PSF over a larger range. FIG. 5 also provides a third graph **503** and a fourth graph **504** showing that masks such as described herein can reduce the error rate (i.e., the number of mismatches between produced basecalls and the reference genome) and increase the throughput in simulations where all known noise sources were added to PSFs generated using optics with masks and the associated images were then used for sequencing a reference genome.

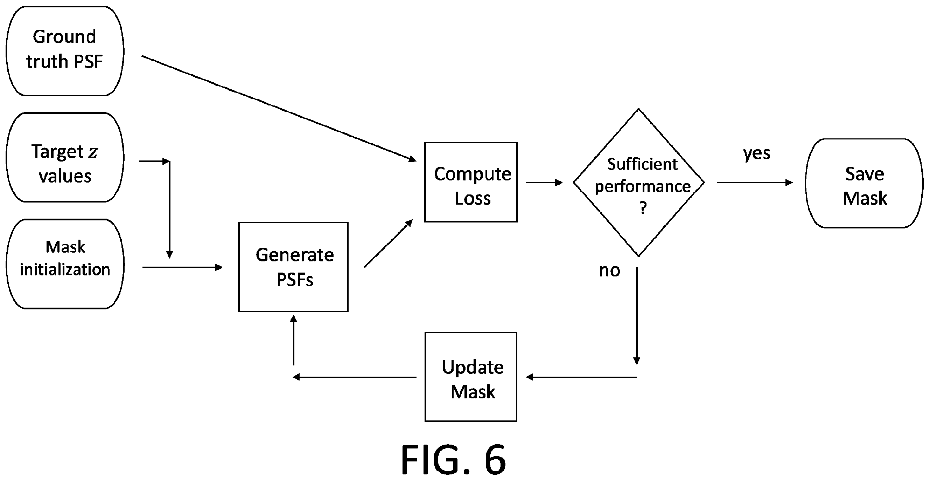

Turning now to FIG. 6, that figure illustrates a method by which a mask 401 such as shown in FIG. 4 may be designed. This method, which will preferably be performed in simulation using a computer such as shown in FIG. 11. provides an iterative process in which PSFs generated based on use of a mask will be compared with a ground truth PSF (e.g., a best possible PSF, which would be a PSF with zero defocus and no mask), and the differences between the ground truth PSF and the mask generated PSFs will be used as feedback for improving the mask. In the method of FIG. 6, the mask may be initialized to have random values (e.g., random phase delay values between 0 and 2π for a phase mask, or random opacity values between 0 and 100% for an amplitude mask) and so the PSFs generated by that mask for the system's target z values (i.e., the defocus amounts which could be expected to be encountered in practice) would be expected to provide little, if any, improvement relatives to the PSFs that would be generated at the target z values without any mask. However, by calculating a loss of the generated PSFs relative to the ground truth PSF (e.g., using a mean squared error loss function), and then using that loss to modify the mask design (e.g., through gradient descent updating), can gradually improve the performance of the mask being designed until some sufficiency condition (e.g., performance ceases to improve, performance improvement across iterations decreases below a threshold speed, a predetermined number of iterations has been reached, etc.) is met. At which point, the final mask design from when the performance condition was satisfied may be manufactured an incorporated into sequencers such as shown in FIGS. 1 or 2 to extend their depth of field relative to what would otherwise be available.

Of course, it should be understood that the method of FIG. 6 and associated discussion are intended to be illustrative only, and that variations on the depicted method and associated discussion are both contemplated and could be implemented by those of ordinary skill in the art without undue experimentation in light of this disclosure. To illustrate, consider the loss function which is used to generate the feedback for updating the mask. As described in U.S. reissued patent RE48,561, reissued May 18, 2021, in some cases a sample may be disposed on multiple vertically separated surfaces. In such a case, expanding the depth of field may have a drawback in that a depth of field which is great enough to cover multiple imaging surfaces can result in signals from the multiple surfaces interfering with each other. To address this issue, in some implementations, rather than simply using a loss function such as mean squared error, a loss function may be defined in a piecewise manner, with different portions of the piecewise function corresponding target z values at which signals from different surfaces would be detected. Other modifications to the loss function which may be used in various implementations are also possible. For instance, in a case where the mask is an amplitude mask, the mask may be designed by randomly assigning clusters in a simulated sample as "on" or "off," and then using a function such as equation 1 below (in which Iₒₙ and I_{off} are the mean intensities of on and off clusters, respectively, α is a weighting coefficient, and σₒₙ and σ_{off} are, the standard deviation of on and off clusters, respectively) as the loss function in a process such as shown in FIG. 6. $L = - log \left({I}_{on}-{I}_{off}\right) + α \left({σ}_{on}+{σ}_{off}\right)$ Equation 1

Variations are also possible in aspects of a process such as shown in FIG. 6 other than the loss function as discussed above. For example, because different wavelengths of light will generally have different PSFs, mask design may be performed in a way that accounts for the particular wavelengths which will be used in the sequencer where such a mask will be used. This may be done, for example, by training wavelength specific masks (e.g., phase masks), rather than by designing a single mask that would be used for all wavelengths. Subsequently, these multiple masks could be placed at or close to the Fourier plane (e.g., immediately behind the objective) before the camera of the sequencer of each channel of the detection path. When designing these multiple masks, a median wavelength may be chosen to represent each wavelength which would be read in practice, and masks may be simultaneously designed using each of those median wavelengths. As another variation which may be implemented in some cases to address issues raised by the use of multiple wavelengths, in some cases the dimensions of pixels which would detect different wavelengths may be held constant by changing either the dimensions of the pixels in the image plane, or the number of pixels in the image and pupil planes to allow joint optimization of masks across wavelengths despite the fact that having fixed sensor pixel dimensions implies that there would be frequency dependent pupil pixel dimensions.

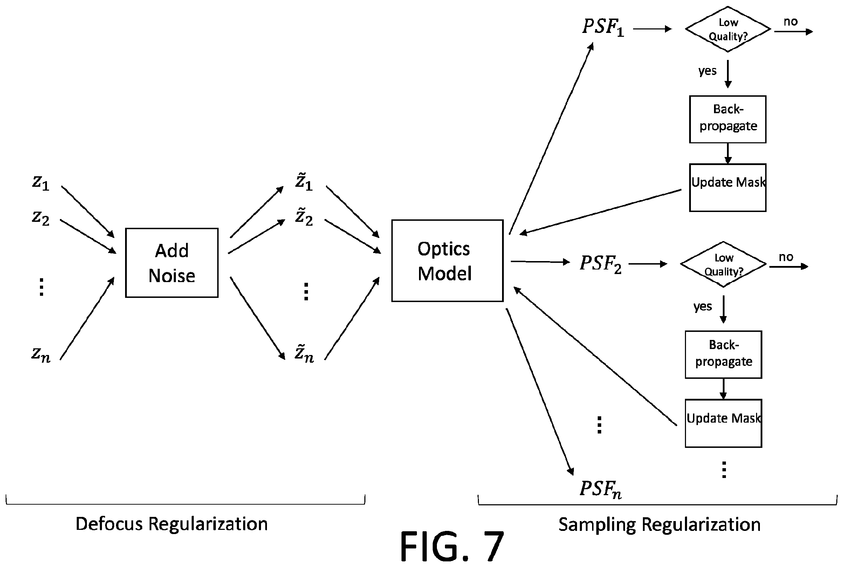

As another example of a variation which may be present in some implementations, consider a situation in which there was limited training data to use in a method such as shown in FIG. 6. In this type of scenario, the effective amount of training data may be increased by various regularization approaches, such as adding noise to defocus values during each forward pass, subsample PSFs for back propagation and weight updates (e.g., if there are 20 PSFs for target z values, use only 3 of the 20, such as the three worst PSFs, for back propagation), and/or adding Gaussian white noise to generated PSFs. An example of a modified design method which illustrates the first two of these potential regularization approaches is provided in FIG. 7.

It should be understood that, while the above examples illustrated that variations are possible within a method such as shown in FIG. 6, a method such as shown in FIG. 6 may not be adhered to in all cases, and other methods may be used when designing masks based on this disclosure. For instance, while a method such as shown in FIG. 6 may start with a pupil function and assume a diffraction model to generate PSFs, in some cases a mask may be designed in an inverse analytical fashion. For example, this may be done using a method which starts with a desired aberration-free in-focus phase mask (either through simulation or experiment), applies the inverse diffraction equation to the masks determined by the pupil function (e.g., taking the inverse Fourier transform in the case of Fraunhofer diffraction), and taking a mean (e.g., a true mean, median, or weighted average) of the resulting pupil functions to arrive at the final mask design. As another example, an inverse analytical approach may be implemented in a method in which a desired depth of field range is selected, the effects of focus aberrations on the pupil plane for every desired z-value are generated, and a circular average (e.g., mean, median or weighted average) is taken of the resulting pupil functions to arrive at the final mask design.

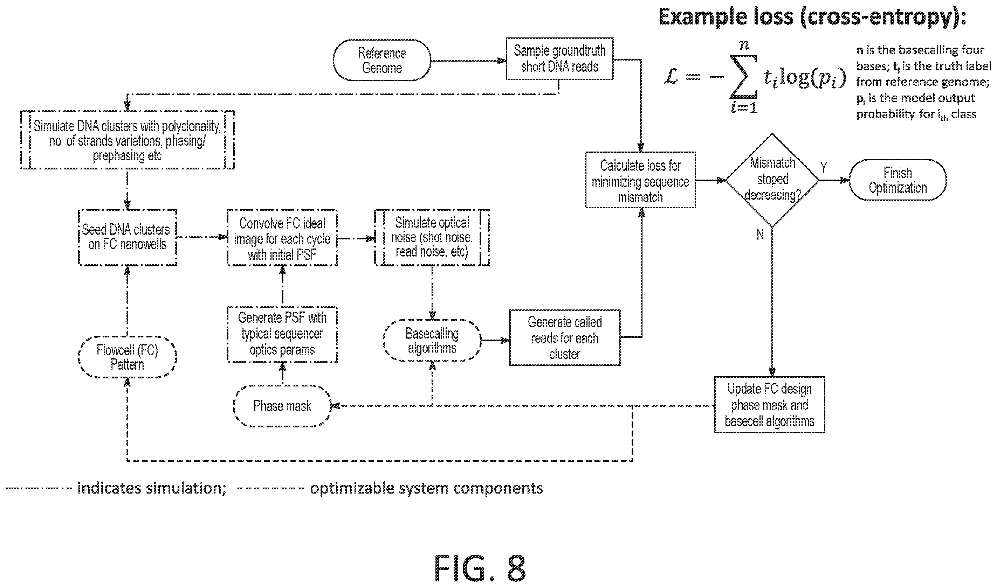

Another example of an alternative design method is provided in FIG. 8, which shows an end to end design process, including an end use of signals processed using a mask (in this case, base calling). In this type of approach, base calling error rate can be used as a loss function, which not only ties mask design to an end use, but also can allow for optimization of the base calling algorithm as well. As shown, this type of approach may begin with generating DNA fragments from a reference genome. These DNA fragments may be seeded on a flowcell according to user-defined flowcell patterns (e.g., pitch, well size, etc.). Noise sources, such as polyclonality, cluster-to-cluster variation, prephasing and phasing may be incorporated, and an image of the flowcell may be added using an optics. The base caller may then be used to base call the flowcell images, and the base calls may be compared to a reference genome to calculate loss. This loss can then be back propagated to update mask parameters and, in some cases, to also update parameters of the base calling algorithm (e.g., where the base calling algorithm is implemented using a machine learning classifier). It should be noted that, while FIG. 8 illustrates this end to end process as being performed to design a phase mask, it is not limited to phase mask design, and other types of masks (e.g., amplitude masks) may also be designed and optimized using the approach illustrated in that figure.

Variations are also possible in aspects of the disclosed technology beyond methods which may be used in designing masks. To illustrate, consider downstream processing of images, and that an image created using a mask may need to be processed by algorithms which had been optimized for images having characteristics other than those produced by using a mask. To address this possibility, in some cases a mask will be integrated along with an image to image translation algorithm (e.g., a neural network, such as described in Ronneberger et al., U-Net: Convolutional Networks for Biomedical Image Segmentation, arXiv: 1505.04597) which had been trained by generating paired images with and without the mask, either in simulation or on a real instrument. As another illustration, consider that system parameters may change over time, for example, due to objective heating, photodamage, or instrument degradation. To address this, in some cases a mask may be integrated in an adaptive manner, such as integrating a phase mask using a deformable mirror or spatial light modulator, thereby allowing for recalibration in the field, and the ability to upgrade without requiring physical replacement of instrument components.

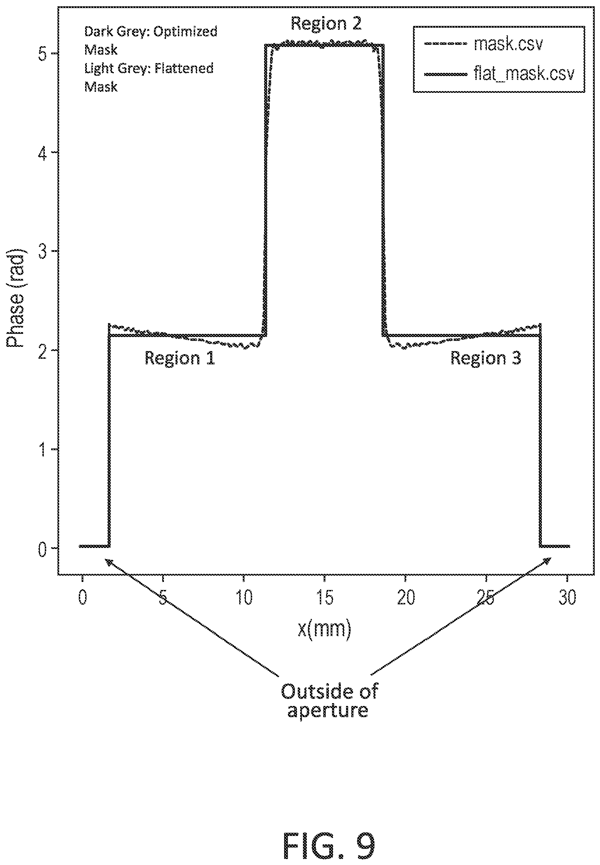

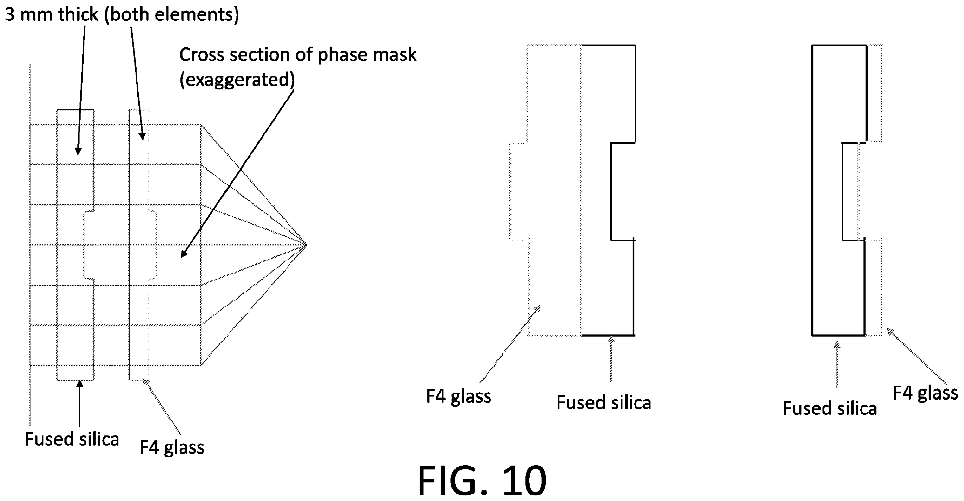

Variations are also possible in how a mask may be manufactured. For example, in some cases application of an iterative design process such as described herein may result in a mask having complex geometry which is difficult to manufacture. To address this, in some cases a mask's geometry may be simplified by dividing the mask into discrete regions. Then, in each region, a median value may be taken for that region (e.g., a median phase value in the case of a phase mask, or a median opacity value in the case of an amplitude mask), and the entire region may be given that value An example of this is shown in FIG. 9, which shows cross sections of both an optimized phase mask, and a phase mask which has been divided into regions and flattened to improve manufacturability. Non-simplifying manufacturing variations may also be utilized in some cases. For instance, to address a scenario in which a mask (e.g., a phase mask) is applied to multiple wavelengths of light, the mask may be manufactured from multiple materials (e.g., one for each peak wavelength of light expected to be emitted from a sample) to minimize phase error caused by the multiple wavelengths. This is illustrated in FIG. 10, which shows how a phase mask can be created using a combination of fused silica and F4 glass to provide ideal phase delay for 588 nm and 440 nm light. Of course, it should be understood that the fused silica and F4 glass illustrated in FIG. 10 are intended to be illustrative only, and that other types of materials (e.g., MgF2, tiO2 glass, etc.) with reasonable differences in dispersion (e.g., a difference of about 5% or greater between materials) could be used in addition to, or as alternatives to, the fused silica and F4 glass shown in FIG. 10.

FIG. 11 illustrates an example computing component that may be used to implement various features of the system and methods disclosed herein, such as the aforementioned features and functionality of one or more aspects of the methods illustrated in FIGS. 6-8.

As used herein, the term module may describe a given unit of functionality that may be performed in accordance with one or more implementations of the present application. As used herein, a module may be implemented utilizing any form of hardware, software, or a combination thereof. For example, one or more processors, controllers, ASICs, PLAs, PALs, CPLDs, FPGAs, logical components, software routines or other mechanisms may be implemented to make up a module. In implementation, the various modules described herein may be implemented as discrete modules or the functions and features described may be shared in part or in total among one or more modules. In other words, as may be apparent to one of ordinary skill in the art after reading this description, the various features and functionality described herein may be implemented in any given application and may be implemented in one or more separate or shared modules in various combinations and permutations. Even though various features or elements of functionality may be individually described or claimed as separate modules, one of ordinary skill in the art will understand that these features and functionality may be shared among one or more common software and hardware elements, and such description shall not require or imply that separate hardware or software components are used to implement such features or functionality.

Where components or modules of the disclosed technology may be implemented in whole or in part using software, in one implementation, these software elements may be implemented to operate with a computing or processing module capable of carrying out the functionality described with respect thereto. One such example computing module is shown in FIG. 11. Various implementations are described in terms of this example-computing module **1000.** After reading this description, it will become apparent to a person skilled in the relevant art how to implement the application using other computing modules or architectures.

Referring now to FIG. 11, computing module **1000** may represent, for example, computing or processing capabilities found within desktop, laptop, notebook, and tablet computers; hand-held computing devices (tablets, PDA's, smart phones, cell phones, palmtops, etc.); mainframes, supercomputers, workstations or servers; or any other type of special-purpose or general-purpose computing devices as may be desirable or appropriate for a given application or environment. Computing module **1000** may also represent computing capabilities embedded within or otherwise available to a given device. For example, a computing module may be found in other electronic devices such as, for example, digital cameras, navigation systems, cellular telephones, portable computing devices, modems, routers, WAPs, terminals and other electronic devices that may include some form of processing capability.

Computing module **1000** may include, for example, one or more processors, controllers, control modules, or other processing devices, such as a processor **1004.** Processor **1004** may be implemented using a general-purpose or special-purpose processing engine such as, for example, a microprocessor, controller, or other control logic. In the illustrated example, processor **1004** is connected to a bus **1002,** although any communication medium may be used to facilitate interaction with other components of computing module **1000** or to communicate externally.

Computing module **1000** may also include one or more memory modules, referred to herein as main memory **1008.** For example, preferably random access memory (RAM) or other dynamic memory, may be used for storing information and instructions to be executed by processor **1004.** Main memory **1008** may also be used for storing temporary variables or other intermediate information during execution of instructions to be executed by processor **1004.** Computing module **1000** may likewise include a read only memory ("ROM") or other static storage device coupled to bus **1002** for storing static information and instructions for processor **1004.**

The computing module **1000** may also include one or more various forms of information storage mechanism **1010,** which may include, for example, a media drive **1012** and a storage unit interface **1020.** The media drive **1012** may include a drive or other mechanism to support fixed or removable storage media **1014.** For example, a hard disk drive, a solid state drive, a magnetic tape drive, an optical disk drive, a CD or DVD drive (R or RW), or other removable or fixed media drive may be provided. Accordingly, storage media **1014** may include, for example, a hard disk, a solid state drive, magnetic tape, cartridge, optical disk, a CD, DVD, or Blu-ray, or other fixed or removable medium that is read by, written to or accessed by media drive **1012.** As these examples illustrate, the storage media **1014** may include a computer usable storage medium having stored therein computer software or data.

In alternative implementations, information storage mechanism **1010** may include other similar instrumentalities for allowing computer programs or other instructions or data to be loaded into computing module **1000.** Such instrumentalities may include, for example, a fixed or removable storage unit **1022** and an interface **1020.** Examples of such storage units **1022** and interfaces **1020** may include a program cartridge and cartridge interface, a removable memory (for example, a flash memory or other removable memory module) and memory slot, a PCMCIA slot and card, and other fixed or removable storage units **1022** and interfaces **1020** that allow software and data to be transferred from the storage unit **1022** to computing module **1000.**

Computing module **1000** may also include a communications interface **1024.** Communications interface **1024** may be used to allow software and data to be transferred between computing module **1000** and external devices. Examples of communications interface **1024** may include a modem or softmodem, a network interface (such as an Ethernet, network interface card, WiMedia, IEEE 802.XX or other interface), a communications port (such as for example, a USB port, IR port, RS232 port Bluetooth^{®} interface, or other port), or other communications interface. Software and data transferred via communications interface **1024** may typically be carried on signals, which may be electronic, electromagnetic (which includes optical) or other signals capable of being exchanged by a given communications interface **1024.** These signals may be provided to communications interface **1024** via a channel **1028.** This channel **1028** may carry signals and may be implemented using a wired or wireless communication medium. Some examples of a channel may include a phone line, a cellular link, an RF link, an optical link, a network interface, a local or wide area network, and other wired or wireless communications channels.

In this document, the terms "computer readable medium", "computer usable medium" and "computer program medium" are used to generally refer to non-transitory media, volatile or non-volatile, such as, for example, memory **1008,** storage unit **1022,** and media **1014.** These and other various forms of computer program media or computer usable media may be involved in carrying one or more sequences of one or more instructions to a processing device for execution. Such instructions embodied on the medium, are generally referred to as "computer program code" or a "computer program product" (which may be grouped in the form of computer programs or other groupings). When executed, such instructions may enable the computing module **1000** to perform features or functions of the present application as discussed herein.

Although described above in terms of various implementations and implementations, it should be understood that the various features, aspects and functionality described in one or more of the individual implementations are not limited in their applicability to the particular implementation with which they are described, but instead may be applied, alone or in various combinations, to one or more of the other implementations of the application, whether or not such implementations are described and whether or not such features are presented as being a part of a described implementation. Thus, the breadth and scope of protection provided by this document or any related document should not be limited by any of the above-described implementations.

It should be appreciated that all combinations of the foregoing concepts (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

The terms "substantially" and "about" used throughout this disclosure, including the claims, are used to describe and account for small fluctuations, such as due to variations in processing. For example, they may refer to less than or equal to ±5%, such as less than or equal to ±2%, such as less than or equal to ±1%, such as less than or equal to ±0.5%, such as less than or equal to ±0.2%, such as less than or equal to ±0.1%, such as less than or equal to ±0.05%.

To the extent applicable, the terms "first," "second," "third," etc. herein are merely employed to show the respective objects described by these terms as separate entities and are not meant to connote a sense of chronological order, unless stated explicitly otherwise herein.

Terms and phrases used in this document, and variations thereof, unless otherwise expressly stated, should be construed as open ended as opposed to limiting. As examples of the foregoing: the term "including" should be read as meaning "including, without limitation" or the like; the term "example" is used to provide instances of the item in discussion, not an exhaustive or limiting list thereof; the terms "a" or "an" should be read as meaning "at least one,'' "one or more" or the like; and adjectives such as "preexisting," "traditional," "normal," "standard," "known" and terms of similar meaning should not be construed as limiting the item described to a given time period or to an item available as of a given time, but instead should be read to encompass preexisting, traditional, normal, or standard technologies that may be available or known now or at any time in the future. Likewise, where this document refers to technologies that may be apparent or known to one of ordinary skill in the art, such technologies encompass those apparent or known to the skilled artisan now or at any time in the future.

The presence of broadening words and phrases such as "one or more," "at least," "but not limited to" or other like phrases in some instances shall not be read to mean that the narrower case is intended or required in instances where such broadening phrases may be absent. The use of the term "module" does not imply that the components or functionality described or claimed as part of the module are all configured in a common package. Indeed, any or all of the various components of a module, whether control logic or other components, may be combined in a single package or separately maintained and may further be distributed in multiple groupings or packages or across multiple locations.

Additionally, the various implementations set forth herein are described in terms of block diagrams, flow charts and other illustrations. As will become apparent to one of ordinary skill in the art after reading this document, the illustrated implementations and their various alternatives may be implemented without confinement to the illustrated examples. For example, block diagrams and their accompanying description should not be construed as mandating a particular architecture or configuration.

While various implementations of the present disclosure have been described above, it should be understood that they have been presented by way of example only, and not of limitation. Likewise, the various diagrams may depict an example architectural or other configuration for the disclosure, which is done to aid in understanding the features and functionality that may be included in the disclosure. The disclosure is not restricted to the illustrated example architectures or configurations, but the desired features may be implemented using a variety of alternative architectures and configurations. Indeed, it will be apparent to one of skill in the art how alternative functional, logical or physical partitioning and configurations may be implemented to implement the desired features of the present disclosure. Also, a multitude of different constituent module names other than those depicted herein may be applied to the various partitions. Additionally, with regard to flow diagrams, operational descriptions and method claims, the order in which the acts are presented herein shall not mandate that various implementations be implemented to perform the recited functionality in the same order unless the context dictates otherwise. The invention is only limited by the appended claims.

## Claims

1. A method of extending a depth of field of a nucleic acid sequencer (200, 500), the method comprising:
performing a set of optimization steps comprising:
determining a first result, wherein the first result is a result of passing light emitted by a sample at a nucleic acid site (250, 310) through an objective lens (235) and a mask (401), wherein the objective lens (235) is focused on a plane which is separated from the nucleic acid site (250, 310) by a target defocus amount, and wherein the mask (401) is disposed between the objective lens (235) and a set of detection pixels;
determining whether there is a discrepancy between the first result and a second result, wherein the second result is an ideal result for detecting light emitted by the sample at the nucleic acid site (250, 310); and
based on determining there is the discrepancy between the first result and the second result, performing a set of updating tasks comprising updating the mask (401) based on the determined discrepancy between the first result and the second result; and
repeating the set of optimization steps one or more times.

2. The method of claim 1, wherein:
the first result is a first point spread function (PSF) indicating how light emitted by the sample at the nucleic acid site (250, 310) is detected by the set of detection pixels (221, 540) after passing through the objective lens (235) and the mask (401) when the objective lens (235) is out of focus by a target defocus amount; and
the second result is a second PSF indicating how light emitted by the sample at the nucleic acid site (250, 310) is detected by the set of detection pixels (221, 540) when the sample at the nucleic acid site (250, 310) is focused on a surface of the sample.

3. The method of claim 2, wherein:
the nucleic acid sequencer (200, 500) is configured to detect light signals emitted from multiple surfaces of a multi-surface flowcell (510);
updating the mask (401) based on the determined discrepancy comprises applying a loss function, wherein the piecewise loss function comprises:
a first loss component configured to ensure that signals from a first surface of the multi-surface flowcell (510) are in focus; and
a second loss component configured to ensure that signals from a second surface of the multi-surface flowcell (510) are out of focus.

4. The method of claim 1, wherein:
the mask (401) is an amplitude mask;
the sample at the nucleic acid site (250, 310) comprises a plurality of clusters;
the set of optimization steps comprises:
separating the plurality of clusters into a first set of clusters and a second set of clusters; and
updating the mask (401) based on the determined discrepancy comprises determining a loss based on a mean and a standard deviation of the first set of clusters, and a mean and a standard deviation of the second set of clusters.

5. The method of claim 1, wherein:
the first result is a sequence of base calls generated by a base calling algorithm based on light emitted from the sample at the nucleic acid site (250, 310) and detected by the set of detection pixels (221, 540) after passing through objective lens (235) and the mask (401); and
the second result is a sequence of base calls for a reference genome.

6. The method of claim 5, wherein:
the base calling algorithm comprises a machine learning model; and
the set of updating tasks comprises updating the machine learning model based on the discrepancy between the first result and the second result.

7. The method of claim 1, wherein:
the nucleic acid sequencer (200, 500) is configured to detect light signals in a plurality of wavelengths;
the first result is a result of passing a first wavelength of light emitted by the sample at the nucleic acid site (250, 310) through the objective lens (235) and the mask (401); and
the set of optimization steps comprises:
determining a third result, wherein the third result is a result of passing a second wavelength of light emitted by the sample at the nucleic acid site (250, 310) through the objective lens (235) and a second mask, wherein the objective lens (235) is focused on the plane which is separated from the nucleic acid site (250, 310) by the target defocus amount, and wherein the second mask is disposed between the objective lens (235) and a second set of detection pixels (221, 540);
determining whether there is a discrepancy between the second result and the third result; and
based on determining there is a discrepancy between the second result and the third result, performing a second set of updating tasks comprising updating the second mask based on the determined discrepancy between the second result and the third result.

8. The method of claim 1, wherein the mask (401) is a phase mask.

9. The method of claim 1, wherein, on each repetition of the set of optimization steps:
determining the first result is performed a plurality of times, wherein each time from the plurality of times, the target defocus amount is different from the target defocus amount on each other time from the plurality of times;
performing the set of updating tasks is performed a set of times, wherein each time the set of updating tasks is performed, a loss is calculated which corresponds to the target defocus amount from a different time from the plurality of times.

10. The method of claim 9, wherein, on each repetition of the set of optimization steps, the plurality of times the first result is determined has a cardinality less than the set of times the set of updating tasks is performed.

11. The method of claim 10, wherein, on each repetition of the set of optimization tasks:
the set of updating tasks comprises determining the target defocus resulting in the largest discrepancies between the first result and the second result; and
each loss which is calculated during performance of the set of updating tasks corresponds to a target defocus amount which results in a greater discrepancy between the first result and the second result than any target defocus amount which does not correspond to a loss calculated during that repetition of the set of optimization tasks.

## Patentansprüche

1. Ein Verfahren zum Erweitern einer Schärfentiefe eines Nukleinsäure-Sequenzers (200, 500), wobei das Verfahren Folgendes beinhaltet:
Durchführen eines Satzes von Optimierungsschritten, die Folgendes beinhalten:
Bestimmen eines ersten Ergebnisses, wobei das erste Ergebnis ein Ergebnis des Durchleitens von Licht, das von einer Probe an einer Nukleinsäurestelle (250, 310) emittiert wird, durch eine Objektivlinse (235) und eine Maske (401) ist, wobei die Objektivlinse (235) auf eine Ebene fokussiert ist, die von der Nukleinsäurestelle (250, 310) durch einen Zieldefokusbetrag getrennt ist, und wobei die Maske (401) zwischen der Objektivlinse (235) und einem Satz von Detektionspixeln angeordnet ist;
Bestimmen, ob es eine Diskrepanz zwischen dem ersten Ergebnis und einem zweiten Ergebnis gibt, wobei das zweite Ergebnis ein ideales Ergebnis zum Detektieren von Licht ist, das von der Probe an der Nukleinsäurestelle (250, 310) emittiert wird; und
basierend auf dem Bestimmen, dass es die Diskrepanz zwischen dem ersten Ergebnis und dem zweiten Ergebnis gibt, Durchführen eines Satzes von Aktualisierungsaufgaben, die das Aktualisieren der Maske (401) basierend auf der bestimmten Diskrepanz zwischen dem ersten Ergebnis und dem zweiten Ergebnis beinhalten; und
Wiederholen des Satzes von Optimierungsschritten ein oder mehrere Male.

2. Verfahren gemäß Anspruch 1, wobei:
das erste Ergebnis eine erste Punktspreizfunktion (PSF) ist, die angibt, wie Licht, das von der Probe an der Nukleinsäurestelle (250, 310) emittiert wird, von dem Satz von Detektionspixeln (221, 540) nach dem Durchleiten durch die Objektivlinse (235) und die Maske (401) detektiert wird, wenn die Objektivlinse (235) um einen Zieldefokusbetrag außer Fokus ist; und
das zweite Ergebnis eine zweite PSF ist, die angibt, wie Licht, das von der Probe an der Nukleinsäurestelle (250, 310) emittiert wird, von dem Satz von Detektionspixeln (221, 540) detektiert wird, wenn die Probe an der Nukleinsäurestelle (250, 310) auf eine Oberfläche der Probe fokussiert ist.

3. Verfahren gemäß Anspruch 2, wobei:
der Nukleinsäure-Sequenzer (200, 500) konfiguriert ist, um Lichtsignale zu detektieren, die von mehreren Oberflächen einer Mehroberflächen-Durchflusszelle (510) emittiert werden;
das Aktualisieren der Maske (401) basierend auf der bestimmten Diskrepanz das Anwenden einer stückweisen Verlustfunktion beinhaltet, wobei die stückweise Verlustfunktion Folgendes beinhaltet:
eine erste Verlustkomponente, die konfiguriert ist, um sicherzustellen, dass Signale von einer ersten Oberfläche der Mehroberflächen-Durchflusszelle (510) im Fokus sind; und
eine zweite Verlustkomponente, die konfiguriert ist, um sicherzustellen, dass Signale von einer zweiten Oberfläche der Mehroberflächen-Durchflusszelle (510) außer Fokus sind.

4. Verfahren gemäß Anspruch 1, wobei:
die Maske (401) eine Amplitudenmaske ist;
die Probe an der Nukleinsäurestelle (250, 310) eine Vielzahl von Clustern beinhaltet;
der Satz von Optimierungsschritten Folgendes beinhaltet:
Trennen der Vielzahl von Clustern in einen ersten Satz von Clustern und einen zweiten Satz von Clustern; und
das Aktualisieren der Maske (401) basierend auf der bestimmten Diskrepanz das Bestimmen eines Verlusts basierend auf einem Mittelwert und einer Standardabweichung des ersten Satzes von Clustern und einem Mittelwert und einer Standardabweichung des zweiten Satzes von Clustern beinhaltet.

5. Verfahren gemäß Anspruch 1, wobei:
das erste Ergebnis eine Sequenz von Basenaufrufen ist, die von einem Basenaufrufalgorithmus basierend auf Licht erzeugt wird, das von der Probe an der Nukleinsäurestelle (250, 310) emittiert wird und von dem Satz von Detektionspixeln (221, 540) nach dem Durchleiten durch die Objektivlinse (235) und die Maske (401) detektiert wird; und
das zweite Ergebnis eine Sequenz von Basenaufrufen für ein Referenzgenom ist.

6. Verfahren gemäß Anspruch 5, wobei:
der Basenaufrufalgorithmus ein Modell zum maschinellen Lernen beinhaltet; und
der Satz von Aktualisierungsaufgaben das Aktualisieren des Modells zum maschinellen Lernen basierend auf der Diskrepanz zwischen dem ersten Ergebnis und dem zweiten Ergebnis beinhaltet.

7. Verfahren gemäß Anspruch 1, wobei:
der Nukleinsäure-Sequenzer (200, 500) konfiguriert ist, um Lichtsignale in einer Vielzahl von Wellenlängen zu detektieren;
das erste Ergebnis ein Ergebnis des Durchleitens einer ersten Wellenlänge von Licht, das von der Probe an der Nukleinsäurestelle (250, 310) emittiert wird, durch die Objektivlinse (235) und die Maske (401) ist; und
der Satz von Optimierungsschritten Folgendes beinhaltet:
Bestimmen eines dritten Ergebnisses, wobei das dritte Ergebnis ein Ergebnis des Durchleitens einer zweiten Wellenlänge von Licht, das von der Probe an der Nukleinsäurestelle (250, 310) emittiert wird, durch die Objektivlinse (235) und eine zweite Maske ist, wobei die Objektivlinse (235) auf die Ebene fokussiert ist, die von der Nukleinsäurestelle (250, 310) durch den Zieldefokusbetrag getrennt ist, und wobei die zweite Maske zwischen der Objektivlinse (235) und einem zweiten Satz von Detektionspixeln (221, 540) angeordnet ist;
Bestimmen, ob es eine Diskrepanz zwischen dem zweiten Ergebnis und dem dritten Ergebnis gibt; und
basierend auf dem Bestimmen, dass es eine Diskrepanz zwischen dem zweiten Ergebnis und dem dritten Ergebnis gibt, Durchführen eines zweiten Satzes von Aktualisierungsaufgaben, die das Aktualisieren der zweiten Maske basierend auf der bestimmten Diskrepanz zwischen dem zweiten Ergebnis und dem dritten Ergebnis beinhalten.

8. Verfahren gemäß Anspruch 1, wobei die Maske (401) eine Phasenmaske ist.

9. Verfahren gemäß Anspruch 1, wobei bei jeder Wiederholung des Satzes von Optimierungsschritten:
Bestimmen, dass das erste Ergebnis eine Vielzahl von Malen bestimmt wird, wobei jedes Mal aus der Vielzahl von Malen der Zieldefokusbetrag von dem Zieldefokusbetrag bei jedem anderen Mal aus der Vielzahl von Malen verschieden ist;
Durchführen des Satzes von Aktualisierungsaufgaben einen Satz von Malen, wobei jedes Mal, wenn der Satz von Aktualisierungsaufgaben durchgeführt wird, ein Verlust berechnet wird, der dem Zieldefokusbetrag von einem anderen Mal aus der Vielzahl von Malen entspricht.

10. Verfahren gemäß Anspruch 9, wobei bei jeder Wiederholung des Satzes von Optimierungsschritten die Vielzahl von Malen, bei denen das erste Ergebnis bestimmt wird, eine Kardinalität aufweist, die geringer ist als eine Kardinalität des Satzes von Malen, bei denen der Satz von Aktualisierungsaufgaben durchgeführt wird.

11. Verfahren gemäß Anspruch 10, wobei bei jeder Wiederholung des Satzes von Optimierungsaufgaben:
der Satz von Aktualisierungsaufgaben das Bestimmen des Zieldefokus beinhaltet, der zu den größten Diskrepanzen zwischen dem ersten Ergebnis und dem zweiten Ergebnis führt; und
jeder Verlust, der während der Durchführung des Satzes von Aktualisierungsaufgaben berechnet wird, einem Zieldefokusbetrag entspricht, der zu einer größeren Diskrepanz zwischen dem ersten Ergebnis und dem zweiten Ergebnis führt als jeder Zieldefokusbetrag, der keinem während dieser Wiederholung des Satzes von Optimierungsaufgaben berechneten Verlust entspricht.

## Revendications

1. Un procédé d'extension d'une profondeur de champ d'un séquenceur d'acide nucléique (200, 500), le procédé comprenant :
la réalisation d'un ensemble d'étapes d'optimisation comprenant :
la détermination d'un premier résultat, le premier résultat étant un résultat de lumière passante émise par un échantillon au niveau d'un site d'acide nucléique (250, 310) à travers une lentille d'objectif (235) et un masque (401), la lentille d'objectif (235) étant focalisée sur un plan qui est séparé du site d'acide nucléique (250, 310) d'une quantité de défocalisation cible, et le masque (401) étant disposé entre la lentille d'objectif (235) et un ensemble de pixels de détection ;
la détermination s'il existe un manque de concordance entre le premier résultat et un deuxième résultat, le deuxième résultat étant un résultat idéal pour détecter la lumière émise par l'échantillon au niveau du site d'acide nucléique (250, 310) ; et
sur la base de la détermination qu'il existe le manque de concordance entre le premier résultat et le deuxième résultat, la réalisation d'un ensemble de tâches de mise à jour comprenant la mise à jour du masque (401) sur la base du manque de concordance déterminé entre le premier résultat et le deuxième résultat ; et
la répétition de l'ensemble d'étapes d'optimisation une ou plusieurs fois.

2. Le procédé de la revendication 1, dans lequel :
le premier résultat est une première fonction d'étalement ponctuel (PSF) indiquant comment la lumière émise par l'échantillon au niveau du site d'acide nucléique (250, 310) est détectée par l'ensemble de pixels de détection (221, 540) après être passée à travers la lentille d'objectif (235) et le masque (401) lorsque la lentille d'objectif (235) est défocalisée d'une quantité de défocalisation cible ; et
le deuxième résultat est une deuxième PSF indiquant comment la lumière émise par l'échantillon au niveau du site d'acide nucléique (250, 310) est détectée par l'ensemble de pixels de détection (221, 540) lorsque l'échantillon au niveau du site d'acide nucléique (250, 310) est focalisé sur une surface de l'échantillon.

3. Le procédé de la revendication 2, dans lequel :
le séquenceur d'acide nucléique (200, 500) est configuré pour détecter des signaux lumineux émis depuis de multiples surfaces d'une cellule d'écoulement à surfaces multiples (510) ;
la mise à jour du masque (401) sur la base du manque de concordance déterminé comprend l'application d'une fonction de perte, la fonction de perte par morceaux comprenant :
une première composante de perte configurée pour s'assurer que des signaux provenant d'une première surface de la cellule d'écoulement à surfaces multiples (510) sont focalisés ; et
une deuxième composante de perte configurée pour s'assurer que des signaux provenant d'une deuxième surface de la cellule d'écoulement à surfaces multiples (510) sont défocalisés.

4. Le procédé de la revendication 1, dans lequel :
le masque (401) est un masque d'amplitude ;
l'échantillon au niveau du site d'acide nucléique (250, 310) comprend une pluralité de groupes ;
l'ensemble d'étapes d'optimisation comprend :
la séparation de la pluralité de groupes en un premier ensemble de groupes et un deuxième ensemble de groupes ; et
la mise à jour du masque (401) sur la base du manque de concordance déterminé comprend la détermination d'une perte sur la base d'une moyenne et
d'un écart-type du premier ensemble de groupes, et d'une moyenne et d'un écart-type du deuxième ensemble de groupes.

5. Le procédé de la revendication 1, dans lequel :
le premier résultat est une séquence d'appels de base générés par un algorithme d'appel de base sur la base de lumière émise depuis l'échantillon au niveau du site d'acide nucléique (250, 310) et détectée par l'ensemble de pixels de détection (221, 540) après être passée à travers la lentille d'objectif (235) et le masque (401) ; et
le deuxième résultat est une séquence d'appels de base pour un génome de référence.

6. Le procédé de la revendication 5, dans lequel :
l'algorithme d'appel de base comprend un modèle d'apprentissage machine ; et
l'ensemble de tâches de mise à jour comprend la mise à jour du modèle d'apprentissage machine sur la base du manque de concordance entre le premier résultat et le deuxième résultat.

7. Le procédé de la revendication 1, dans lequel :
le séquenceur d'acide nucléique (200, 500) est configuré pour détecter des signaux lumineux dans une pluralité de longueurs d'onde ;
le premier résultat est un résultat du fait de laisser passer une première longueur d'onde de lumière émise par l'échantillon au niveau du site d'acide nucléique (250, 310) à travers la lentille d'objectif (235) et le masque (401) ; et
l'ensemble d'étapes d'optimisation comprend :
la détermination d'un troisième résultat, le troisième résultat étant un résultat du fait de laisser passer une deuxième longueur d'onde de lumière émise par l'échantillon au niveau du site d'acide nucléique (250, 310) à travers la lentille d'objectif (235) et un deuxième masque, la lentille d'objectif (235) étant focalisée sur le plan qui est séparé du site d'acide nucléique (250, 310) de la quantité de défocalisation cible, et le deuxième masque étant disposé entre la lentille d'objectif (235) et un deuxième ensemble de pixels de détection (221, 540) ;
la détermination s'il existe un manque de concordance entre le deuxième résultat et le troisième résultat ; et
sur la base de la détermination qu'il existe un manque de concordance entre le deuxième résultat et le troisième résultat, la réalisation d'un deuxième ensemble de tâches de mise à jour comprenant la mise à jour du deuxième masque sur la base du manque de concordance déterminé entre le deuxième résultat et le troisième résultat.

8. Le procédé de la revendication 1, dans lequel le masque (401) est un masque de phase.

9. Le procédé de la revendication 1, dans lequel, à chaque répétition de l'ensemble d'étapes d'optimisation :
la détermination que le premier résultat est réalisé une pluralité de fois, à chaque fois de la pluralité de fois, la quantité de défocalisation cible étant différente de la quantité de défocalisation cible à chaque autre fois de la pluralité de fois ;
la réalisation de l'ensemble de tâches de mise à jour est réalisée un ensemble de fois, à chaque fois que l'ensemble de tâches de mise à jour est réalisé, une perte étant calculée qui correspond à la quantité de défocalisation cible d'une fois différente de la pluralité de fois.

10. Le procédé de la revendication 9, dans lequel, à chaque répétition de l'ensemble d'étapes d'optimisation, la pluralité de fois où le premier résultat est déterminé a une cardinalité inférieure à l'ensemble de fois où l'ensemble de tâches de mise à jour est réalisé.

11. Le procédé de la revendication 10, dans lequel, à chaque répétition de l'ensemble de tâches d'optimisation :
l'ensemble de tâches de mise à jour comprend la détermination de la défocalisation cible résultant en les manques de concordance les plus importants entre le premier résultat et le deuxième résultat ; et
chaque perte qui est calculée pendant la réalisation de l'ensemble de tâches de mise à jour correspond à une quantité de défocalisation cible qui résulte en un plus grand manque de concordance entre le premier résultat et le deuxième résultat que n'importe quelle quantité de défocalisation cible qui ne correspond pas à une perte calculée pendant cette répétition de l'ensemble de tâches d'optimisation.
